(19) Europäisches Patentamt
European Patent Office
Office européen des brevets

(11) Veröffentlichungsnummer: **0 081 730**
**A1**

(12) # EUROPÄISCHE PATENTANMELDUNG

(21) Anmeldenummer: **82110970.9**

(22) Anmeldetag: **27.11.82**

(51) Int. Cl.³: **C 07 D 277/34**, C 07 D 277/68, C 07 D 417/12, C 07 D 257/04, C 07 D 263/38, C 07 D 263/58, C 07 D 271/06, C 07 D 271/10, C 07 D 285/08, C 07 D 285/12

(30) Priorität: **10.12.81 DE 3148839**

(43) Veröffentlichungstag der Anmeldung: **22.06.83**
**Patentblatt 83/25**

(84) Benannte Vertragsstaaten: **BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Diehr, Hans-Joachim, Dr., Höhe 35, D-5600 Wuppertal 1 (DE)**
Erfinder: **Priesnitz, Uwe, Dr., Severinstrasse 58, D-5650 Solingen 1 (DE)**

(54) **Verfahren zur Herstellung von Hetaryloxyacetamiden.**

(57) Herbizid wirksame Hetaryloxyacetamide der allgemeinen Formel I

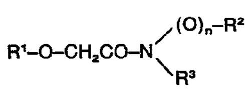

(I)

erhält man in nahezu quantitativen Ausbeuten, indem man Hydroxyacetamide der allgemeinen Formel II

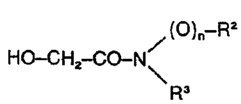

(II)

mit Halogen-hetarenen der allgemeinen Formel III

$R^1$–Hal (III)

in Gegenwart von festem, wasserfreiem Kaliumhydroxid und in Gegenwart eines aprotischen Verdünnungsmittels bei Temperaturen zwischen −50 und +50 °C umsetzt.

In den Formeln (I), (II) und (III) haben die Reste $R^1$, $R^2$, $R^3$ bzw. Hal und der Index n die in der Beschreibung angegebenen Bedeutungen.

EP 0 081 730 A1

BAYER AKTIENGESELLSCHAFT 5090 Leverkusen, Bayerwerk

Zentralbereich
Patente, Marken und Lizenzen Bi/AB
IVa

## Verfahren zur Herstellung von Hetaryloxyacetamiden

Die Erfindung betrifft ein neues Verfahren zur Herstellung von weitgehend bekannten Hetaryloxyacetamiden, welche als Herbizide verwendet werden können.

Es ist bereits bekannt geworden, daß man bestimmte Hetaryloxyacetamide erhält, wenn man Hydroxyacetamide mit Halogen-hetarenen in Gegenwart von Kaliumhydroxid in Isopropanol, von Kaliumcarbonat in Acetonitril, von Kalium-tert-butanolat in tert-Butanol oder von Calciumoxid in Dimethylsulfoxid umsetzt (vergleiche DE-OS 2 914 oo3/ Le A 19 576).

Ausbeute und Qualität der auf diese Weise hergestellten Produkte sind jedoch in vielen Fällen nicht zufriedenstellend.

Es wurde nun ein neues Verfahren zur Herstellung von Hetaryloxyacetamiden der Formel I

$$R^1\text{-}O\text{-}CH_2\text{-}CO\text{-}N\begin{matrix}\diagup (O)_n\text{-}R^2\\ \diagdown R^3\end{matrix} \qquad (I)$$

in welcher

$R^1$ für einen fünfgliedrigen Heterocyclus steht, welcher ein Sauerstoff- oder ein Schwefelatom und zusätzlich 1 bis 3 Stickstoffatome enthält und welcher gegebenenfalls substituiert ist durch Halogen, Cyano, Nitro, Amino, Alkylamino, Arylamino, Dialkylamino, Alkylcarbonylamino, Alkylcarbonyl, Carboxy, Alkoxycarbonyl, Carbamoyl, Alkylaminocarbonyl, Dialkylaminocarbonyl, gegebenenfalls durch Halogen, Nitro oder Alkyl substituiertes Arylaminocarbonyl, gegebenenfalls durch Halogen, Cyano, Nitro, Alkyl oder Alkoxy substituiertes Aryl, gegebenenfalls durch Halogen substituiertes Aralkyl, gegebenenfalls durch Halogen substituiertes Alkoxy, Alkenoxy, Alkinoxy, Alkoxycarbonylalkoxy, Aralkoxy oder Aryloxy, gegebenenfalls durch Halogen substituiertes Alkylthio, Alkenylthio, Alkinylthio, Alkoxycarbonylalkylthio, Aralkylthio oder Arylthio, gegebenenfalls durch Halogen substituiertes Alkylsulfinyl oder Alkylsulfonyl, gegebenenfalls durch Halogen substituiertes Alkyl, Alkenyl, Alkinyl, Alkoxyalkyl, Aralkoxyalkyl, Aryloxyalkyl, Alkylthioalkyl, Alkylsulfinylalkyl, Alkylsulfonylalkyl, Arylthioalkyl, Arylsulfinylalkyl, Arylsulfonylalkyl, Carboxyalkyl, oder Alkoxycarbonylalkyl, gegebenenfalls substituiertes Aminocarbonylalkyl, Cyanoalkyl oder Cycloalkyl, oder welcher gegebenenfalls benzanneliert ist, wobei der Benzo-Rest gegebenenfalls durch Halogen, Alkyl, Halogenalkyl, Alkoxy, Halogenalkoxy, Alkylthio, Halogenalkylthio, Amino, Alkylamino, Dialkylamino, Nitro, Cyano, Alkoxycarbonyl oder durch gegebenenfalls halogen-substituiertes Alkylendioxy substituiert ist,

oder in welcher

$R^1$ für einen durch Phenyl substituierten Tetrazolylrest steht, wobei der Phenylrest gegebenenfalls durch Halogen, Cyano, Nitro und/oder durch einen gegebenenfalls halogen-substituierten Rest aus der Reihe Alkyl, Alkoxy, Alkylthio und Alkylendioxy substituiert ist,

in welcher weiter

n für Null oder 1 steht und

$R^2$ und $R^3$, welche gleich oder verschieden sein können, einzeln für gegebenenfalls substituierte Reste aus der Reihe Alkyl, Alkenyl, Alkinyl, Cycloalk(en)yl, Aralkyl oder Aryl stehen oder - für den Fall, daß n für Null steht - zusammen mit dem Stickstoffatom, an das sie gebunden sind, einen gegebenenfalls substituierten, gegebenenfalls teilweise ungesättigten und gegebenenfalls benzannellierten Mono- oder Bicyclus bilden, der gegebenenfalls weitere Heteroatome enthält,

gefunden, welches dadurch gekennzeichnet ist, daß man Hydroxyacetamide der Formel II

$$HO-CH_2-CO-N\begin{matrix} \diagup (O)_n-R^2 \\ \diagdown R^3 \end{matrix} \qquad (II)$$

in welcher

n, $R^2$ und $R^3$ die oben angegebene Bedeutung haben,

mit Halogen-hetarenen der Formel III

$$R^1-Hal \qquad (III)$$

in welcher

$R^1$ die oben angegebene Bedeutung hat und

Hal für Fluor, Chlor, Brom oder Iod steht,

in Gegenwart von festem, wasserfreiem Kaliumhydroxid und in Gegenwart eines aprotischen Verdünnungsmittels bei Temperaturen zwischen -5o und +5o°C umsetzt.

Es ist als überraschend anzusehen, daß man bei Verwendung von festem Kaliumhydroxid, welches im aprotischen Medium praktisch unlöslich ist, die Hetaryloxyacetamide der Formel (I) in nahezu quantitativen Ausbeuten und in hoher Reinheit erhält.

Verwendet man als Ausgangsstoffe beispielsweise Hydroxyessigsäuredimethylamid und 2-Brombenzthiazol, so kann der Reaktionsverlauf beim erfindungsgemäßen Verfahren durch folgendes Formelschema skizziert werden:

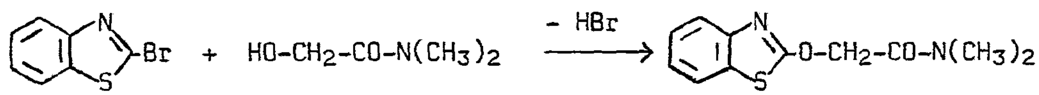


Die als Ausgangsstoffe zu verwendenden Hydroxyacetamide sind durch Formel (II) definiert. Vorzugsweise stehen darin

n für Null oder 1,

$R^2$ für Alkyl, Alkoxyalkyl, Alkenyl oder Alkinyl, jeweils mit bis zu lo Kohlensotffatomen und - für den Fall, daß n für Null steht - auch für Cyanoalkyl oder Alkylthioalkyl, jeweils mit bis zu lo Kohlenstoffatomen, für Cycloalkyl mit 3 bis 12 Kohlenstoffatomen, für gegebenenfalls halogen-substituiertes Benzyl oder Phenethyl oder für Phenyl, welches gegebenenfalls durch gegebenenfalls halogen-substituierte Reste aus der Reihe $C_1$-$C_4$-Alkyl, $C_1$-$C_4$-Alkoxy oder $C_1$-$C_4$-Alkylthio substituiert ist,

$R^3$ für Alkyl, Alkenyl, Alkinyl, Alkoxyalkyl, Alkylthioalkyl, Cyanoalkyl, jeweils mit bis zu lo Kohlenstoffatomen, für Cycloalkyl mit 3 bis 12 Kohlenstoffatomen, für gegebenenfalls halogen-substituiertes Benzyl, Phenethyl oder Naphthyl oder für Phenyl, welches gegebenenfalls durch Halogen, Cyano, Nitro oder durch gegebenenfalls halogen-substituierte Reste aus der Reihe $C_1$-$C_4$-Alkyl, $C_1$-$C_4$-Alkoxy oder $C_1$-$C_4$-Alkylthio substituiert ist,

oder die Reste

$R^2$ und $R^3$ bilden - für den Fall, daß n für Null steht - zusammen mit dem Stickstoffatom, an das sie gebunden sind, einen gegebenenfalls durch 1 bis 3 Alkylgruppen mit jeweils 1 bis 5 Kohlenstoffatomen substituierten, gesättigten oder teilweise ungesättigten und/oder benzannellierten Monocyclus oder Bicyclus mit bis zu 15 Kohlenstoffatomen, welcher gegebenenfalls Sauerstoff als weiteres Heteroatom enthält.

Als Ausgangsstoffe besonders bevorzugt sind diejenigen Verbindungen der Formel (II), in welcher

n für Null oder 1 steht,

$R^2$ für $C_1$-$C_6$-Alkyl, $C_1$-$C_4$-Alkoxy-ethyl, Allyl, Propargyl, 1-Methylpropargyl oder 1,1-Dimethylpropargyl oder - für den Fall, daß n für Null steht - auch für Cyanoethyl, Cyclopentyl, Cyclohexyl, Benzyl oder Phenyl steht, in welcher weiter

$R^3$ für $C_1$-$C_6$-Alkyl, $C_1$-$C_4$-Alkoxy-ethyl, Allyl, Propargyl, 1-Methylpropargyl, 1,1-Dimethyl-propargyl, Cyanoethyl, Cyclopentyl, Cyclohexyl, Benzyl, Naphthyl oder Phenyl steht, welches gegebenenfalls durch Methyl, Chlor, Cyano, Nitro oder Methoxy substituiert ist, wobei auch mehrfache und gemischte Substitution durch die genannten Reste möglich ist, in welcher weiter - für den Fall, daß n für Null steht - die Reste $R^2$ und $R^3$ zusammen mit dem Stickstoffatom, an das sie gebunden sind, für Pyrrolidyl, Monoalkyl- oder Dialkyl-pyrrolidyl mit 1 bis 3 Kohlenstoffatomen je Alkylgruppe, Morpholinyl oder Dialkylmorpholinyl mit 1 bis 3 Kohlenstoffatomen je Alkylgruppe, Piperidyl, Monoalkyl-, Dialkyl- oder Trialkylpiperidyl mit 1 bis 3 Kohlenstoffatomen je Alkylgruppe, ferner für Perhydroazepinyl (Hexamethylenimino-Rest), Trimethyl-perhydroazepinyl, für den Heptamethylenimino-Rest, für den Dodekamethylenimino-Rest, für Indolinyl, für Monoalkyl-, Dialkyl- oder Trialkyl-indolinyl mit bis zu 3 Kohlenstoffatomen je Alkylgruppe, für Perhydroindolyl, Monoalkyl-, Dialkyl- oder Trialkylperhydroindolyl mit 1 bis 3 Kohlenstoffatomen je Alkylgruppe, 1,2,3,4-Tetrahydrochinolyl oder 1,2,3,4-Tetrahydro-iso-chinolyl, Monoalkyl-,Dialkyl- und Trialkyl-1,2,3,4-tetrahydrochinolyl oder -iso-chinolyl mit 1 bis 3 Kohlenstoffatomen je Alkylgruppe, für Perhydrochinolyl oder Perhydro-iso-chinolyl, Monoalkyl-, Dialkyl- oder Trialkylperhydrochinolyl oder -perhydroisochinolyl mit 1 bis 3 Kohlenstoffatomen je Alkylgruppe stehen.

Als Beispiele für die Ausgangsverbindungen der Formel (II) seien genannt:
N-Methoxy-N-methyl-, N-Ethoxy-N-methyl-, N-n-Propoxy-N-methyl-, N-iso-Propoxy-N-methyl-, N-Ethoxy-N-ethyl-, N-n-Propoxy-N-ethyl-, N-iso-Propoxy-N-ethyl-, N-n-Propoxy-N-n-propyl-, N-iso-Propoxy-N-isopropyl-, N-iso-Propoxy-N-n-propyl-, N-Methoxy-N-ethyl-, N-Methoxy-N-n-propyl-, N-Methoxy-N-isopropyl-, N-Methoxy-N-n-butyl-, N-Methoxy-N-isobutyl-, N-Methoxy-N-sec-butyl-, N-Methoxy-N-sec-hexyl-, N-Ethoxy-N-n-propyl-, N-Ethoxy-N-isopropyl-, N-(2-Ethoxy-ethoxy)-N-methyl-, N-(2-Ethoxy-

ethoxy)-N-ethyl-, N-(2-Ethoxy-ethoxy)-N-n-propyl-, N-(2-Ethoxy-ethoxy)-N-isopropyl-, N-(2-Ethoxy-ethoxy)-N-cyclohexyl-, N-Allyloxy-N-allyl-, N-Allyloxy-N-methyl-, N-Allyloxy-N-ethyl-, N-Allyloxy-N-n-propyl-, N-Allyloxy-N-isopropyl-, N-Allyloxy-N-n-butyl-, N-Allyloxy-N-iso-butyl-, N-Allyloxy-N-sec-butyl-, N-Methoxy-N-cyclopentyl-, N-Methoxy-N-cyclohexyl-, N-Methoxy-N-(2-ethoxy-ethyl), N-Ethoxy-N-(2-ethoxy-ethyl)-, N-(2-Ethoxy-ethoxy)-N-(2-ethoxy-ethyl)- und N-(2-Ethoxy-ethoxy)-N-sec-hexyl-hydroxyessigsäureamid sowie Hydroxyessigsäure-dimethylamid, -diethylamid, -di-n-propylamid, -di-iso-propylamid, -N-methyl-N-iso-propylamid, -N-methyl-N-iso-butylamid, -N-methyl-N-sec-butylamid, -di-(2-ethyl-hexyl)-amid, -N-methyl-N-(2-cyano-ethyl)-amid, -di-(2-methoxy-ethyl)-amid, -di-allylamid, -N-methyl-N-propargylamid, -N-methyl-N-(1-methyl-propargyl)-amid, -dipropargylamid, -N-methyl-N-cyclopentylamid, -N-methyl-N-cyclohexylamid, -N-methyl-anilid, -N-methyl-N-(2-methyl-phenyl)-, -N-methyl-N-(3-methyl-phenyl)-, -N-methyl-N-(4-methyl-phenyl)-amid, -N-methyl-N-(2-chlorphenyl)-, -N-methyl-N-(3-chlorphenyl)-, -N-methyl-N-(4-chlor-phenyl)-amid, -N-methyl-N-(3-nitro-6-methyl-phenyl)-amid, -N-ethyl-anilid, -N-ethyl-N-(2-methyl-phenyl)-, -N-ethyl-N-(3-methylphenyl)-, -N-ethyl-N-(4-methyl-phenyl)-amid, -N-ethyl-N-(2-chlor-phenyl)-, -N-ethyl-N-(3-chlor-phenyl)-, -N-ethyl-N-(4-chlor-phenyl)-amid, -N-ethyl-N-(3-nitro-6-methyl-phenyl)-amid, -N-propylanilid, -N-propyl-N-(2-methyl-phenyl)-, -N-propyl-N-(3-methyl-phenyl)-, -N-propyl-N-(4-methyl-phenyl)-amid, -N-propyl-N-(2-chlor-phenyl)-, -N-propyl-N-(3-chlor-phenyl)-, -N-propyl-N-(4-chlor-phenyl)-amid, -N-iso-propyl-N-(2-methyl-phenyl)-, -N-iso-propyl-N-(3-methyl-phenyl)-, -N-iso-propyl-N-(4-methyl-phenyl)-amid, -N-iso-propyl-N-(3-nitro-6-methyl-phenyl)-amid, -N-butyl-anilid, -N-butyl-N-(2-methyl-phenyl)-, -N-butyl-N-(3-methyl-phenyl)-, -N-butyl-N-(4-methyl-phenyl)-amid, -N-butyl-N-(2-chlor-phenyl)-, -N-butyl-N-(3-chlor-phenyl)-, -N-butyl-N-(4-chlor-phenyl)-amid, -N-isobutyl-N-(2-methyl-phenyl)-, -N-iso-butyl-N-(3-methyl-phenyl)-, -N-iso-butyl-N-(4-methyl-phenyl)-amid,

-N-iso-butyl-N-(3-nitro-6-methyl-phenyl)-amid, -N-methyl-N-naphth(1)-yl-amid, -N-methyl-N-naphth(2)ylamid, -N-ethyl-N-naphth(1)ylamid, -N-ethyl-N-naphth(2)ylamid, -N-n-propyl-N-naphth(2)ylamid, -N-iso-propyl-N-naphth(2)ylamid, -N-n-butyl-N-naphth(2)ylamid,-N-isobutyl-N-naphth(2)ylamid, -dibenzylamid, -N-methyl-N-benzylamid, -N-ethyl-N-benzylamid, -N-propyl-N-benzylamid, -N-butyl-N-benzylamid, -pyrrolidid, -2-methyl-pyrrolidid, -morpholid, -3,5-dimethyl-morpholid-, -piperidid, -2-methyl-piperidid, -4-methyl-piperidid, -2,4-dimethyl-piperidid, -2,4-dimethyl-piperidid, -2,4,6-trimethyl-piperidid, -2-ethyl-piperidid, -4-ethyl-piperidid, -2,4-diethyl-piperidid, -2,4,6-triethyl-piperidid, -2-methyl-4-ethyl-piperidid, -2-ethyl-4-methyl-piperidid, -2-methyl-5-ethyl-piperidid, -2-ethyl-5-methyl-piperidid, -2-methyl-6-ethyl-piperidid, -indolinid, -2-methyl-1,2,3,4-tetrahydrochinolid, -perhydroindolid, -2-methyl-perhydroindolid, -2,2-dimethyl-perhydroindolid, -1,2,3,4-tetrahydrochinolid, -1,2,3,4-tetrahydro-iso-chinolid und -perhydroisochinolid.

Die Hydroxyacetamide der Formel (II) sind bekannt und/oder können nach an sich bekannten Verfahren hergestellt werden (vergleiche EP 55o1 und 18 497 sowie DE-OS 2 9o4 49o und 2 946 524).

Die weiter als Ausgangsstoffe zu verwendenden Halogenhetarene sind durch Formel(III) definiert. Vorzugsweise stehen darin

Hal für Chlor oder Brom und

$R^1$ für den Rest

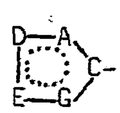

worin

C für Kohlenstoff steht,
A für C-$R^4$ oder N steht,
D für C-$R^5$ oder N steht,
E für C-$R^6$, N, O oder S steht und
G für C-$R^7$, N, O oder S steht,

mit der Maßgabe, daß wenigstens eines der Ringglieder (A, D, E oder G) für N steht und mindestens eines der Ringglieder für O oder S steht,

und wobei die Reste

$R^4$, $R^5$, $R^6$ und $R^7$,

welche gleich oder verschieden sein können, einzeln für Wasserstoff, Halogen, Nitro, Cyano, Amino, $C_1$-$C_4$-Alkylamino, Di-$C_1$-$C_4$-alkylamino, $C_1$-$C_4$-Alkylcarbonylamino, $C_1$-$C_4$-Alkyl-carbonyl, Carboxy, $C_1$-$C_4$-Alkoxycarbonyl, Carbamoyl, $C_1$-$C_4$-Alkylamino-carbonyl, Di-$C_1$-$C_4$-alkyl-amino-carbonyl, für gegebenenfalls durch Halogen, Nitro oder $C_1$-$C_4$-Alkyl substituiertes Phenyl-amino-carbonyl, für gegebenenfalls durch Halogen, Nitro, Cyano, $C_1$-$C_4$-Alkyl oder $C_1$-$C_4$-Alkoxy substituiertes Phenyl, für gegebenenfalls durch Halogen substituiertes Benzyl oder Phenylethyl, für gegebenenfalls durch Halogen substituiertes $C_1$-$C_4$-Alkoxy, $C_2$-$C_4$-Alkenoxy, $C_2$-$C_4$-Alkinoxy, $C_1$-$C_4$-Alkoxy-carbonyl-methoxy, Benzyloxy oder Phenoxy, für gegebenenfalls halogen-substituiertes $C_1$-$C_4$-Alkylthio, $C_2$-$C_4$-Alkenylthio, $C_2$-$C_4$-Alkinylthio, $C_1$-$C_4$-Alkoxy-carbonyl-methylthio, Benzylthio, Phenylthio, $C_1$-$C_4$-Alkylsulfinyl oder $C_1$-$C_4$-Alkylsulfonyl, für gegebenenfalls halogensubstituiertes $C_1$-$C_6$-Alkyl, $C_3$-$C_6$-Alkenyl oder $C_3$-$C_6$-Alkinyl, für Cyano-$C_1$-$C_4$-alkyl, $C_1$-$C_4$-Alkoxy-$C_1$-$C_2$-alkyl, Phenoxy- und Phenylthio-methyl, Benzyloxy- und Benzylthiomethyl, $C_1$-$C_4$-Alkylthio-$C_1$-$C_2$-alkyl, $C_1$-$C_4$-Alkyl- und Phenylsulfinyl-$C_1$-$C_2$-alkyl, $C_1$-$C_4$-Alkyl- und Phenylsulfonyl-$C_1$-$C_2$-alkyl, Carboxy-$C_1$-$C_2$-alkyl, $C_1$-$C_4$-Alkoxy-carbonyl-$C_1$-$C_2$-alkyl,

$C_1$-$C_4$-Alkylaminocarbonyl-$C_1$-$C_2$-alkyl, Di-$C_1$-$C_4$-Alkylaminocarbonyl-$C_1$-$C_2$-alkyl, Phenylaminocarbonyl-$C_1$-$C_2$-alkyl oder $C_3$-$C_{12}$-Cycloalkyl stehen,

oder wobei jeweils zwei benachbarte Reste $R^4$ und $R^5$, oder $R^5$ und $R^6$, oder $R^6$ und $R^7$, zusammen für einen annellierten Benzorest stehen, der durch Halogen, Nitro, Cyano oder durch gegebenenfalls halogensubstituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiert sein kann;

$R^1$ steht ferner vorzugsweise für den Rest

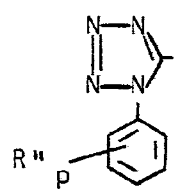

wobei

p für 1 bis 5 steht und

$R''$ für Wasserstoff, Halogen, Cyano,Nitro und/oder für gegebenenfalls halogen-substituierte Reste aus der Reihe $C_1$-$C_4$-Alkyl, $C_1$-$C_4$-Alkoxy, $C_1$-$C_4$-Alkylthio und $C_1$-$C_2$-Alkylendioxy steht.

Als Ausgangsstoffe besonders bevorzugt sind diejenigen Verbindungen der Formel (III), in welcher

Hal für Chlor steht und

$R^1$ für einen der nachstehenden Azolylreste steht:

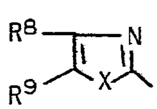 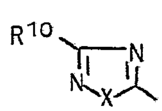 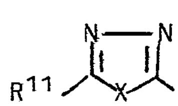 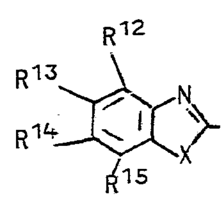

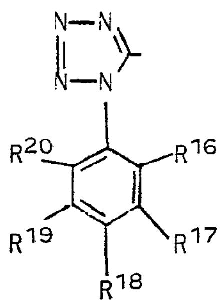

worin

X jweils für Sauerstoff oder Schwefel steht,

die Reste $R^8$, $R^9$, $R^{10}$, $R^{11}$, welche gleich oder verschieden sein können, einzeln für Wasserstoff, Nitro, Cyano, $C_1$-$C_3$-Alkylcarbonyl, $C_1$-$C_3$-Alkoxycarbonyl, gegebenenfalls durch Fluor, Chlor oder Brom, Methyl, Methoxy, Nitro, Amino und/oder Cyano 1- oder 2-fach substituiertes Phenyl, für Phenoxy oder Phenylthio, für $C_1$-$C_3$-Alkylthio oder $C_1$-$C_3$-Alkoxy, für $C_1$-$C_3$-Alkylsulfinyl oder $C_1$-$C_3$-Alkylsulfonyl, für $C_1$-$C_4$-Alkyl, Trifluormethyl, Cyano-$C_1$-$C_4$-alkyl, $C_2$-$C_4$-Alkenyl, Benzyloxymethyl, $C_1$-$C_3$-Alkyl-amino, N-$C_1$-$C_3$-Alkyl-N-$C_1$-$C_4$-alkyl-carbonylamino, Phenoxymethyl-benzylthio, $C_1$-$C_3$-Alkylcarbonyloxy stehen und

die Reste $R^{12}$ bis $R^{20}$, welche gleich oder verschieden sein können, einzeln für Wasserstoff, Brom, Chlor, Nitro, $C_1$-$C_2$-Alkyl, $C_1$-$C_2$-Alkoxy, Trifluormethyl, Trifluormethoxy stehen.

Als Ausgangsstoffe der Formel (III) seien beispielsweise genannt: 2-Chlor- und 2-Brom-oxazol und -thiazol, 2,4-Dichlor-, 2,5-Dichlor- und 2,4,5-Trichlor-oxazol und -thiazol, 4-Methyl-, 5-Methyl-, 4-tert-Butyl-, 4,5-Dimethyl-, 4-Methyl-5-cyano-, 4-Methyl-5-chlor-, 5-Methyl-4-chlor-, 4-Methyl-5-methoxycarbonyl-, 4-Methyl-5-ethoxycarbonyl-, 4-Methyl-5-isopropoxycarbonyl-, 4-Methyl-5-acetyl-, 5-Phenyl-, 4,5-Diphenyl-, 4-Chlor-5-phenyl-, 4-Chlor-5-(3,4-dichlor-phenyl)- und

4-Methyl-5-phenylthio-2-chloroxazol, -2-brom-oxazol, -2-chlor-thiazol und -2-brom-thiazol; 3-tert-Butyl-4-cyano-, 3-But-3-en-1-yl-, 3,4-Bis-ethoxycarbonyl-, 3-Phenyl-, 3-Ethyl-4-phenyl-5-chlor-isoxazol, -5-chlor-isothiazol, -5-brom-isoxazol und -5-brom-isothiazol; 3,5-Bis-ethoxycarbonyl-4-chlor- und 3,5-Bis-ethoxycarbonyl-4-brom-isoxazol und -iso-thiazol; 3,5-Dichlor-1,2,4-oxadiazol, 3-Methyl-, 3-Ethyl-, 3-n-Propyl-, 3-iso-Propyl-, 3-tert-Butyl-, 3-Trifluormethyl-, 3-Trichlormethyl-, 3-Methylthio-, 3-Methylsulfinyl-, 3-Methylsulfonyl- und 3-Benzylthio-5-chlor-1,2,4-thiadiazol und -5-brom-1,2,4-thiadiazol; 4-Methyl-, 4-Ethyl-, 4-n-Propyl- und 4-iso-Propyl-3-chlor-1,2,5-thiadiazol und -3-brom-1,2,5-thiadiazol; 2-Chlor- und 2-Brom-1,3,4-oxadiazol, 2-Chlor- und 2-Brom-5-phenyl-1,3,4-thiadiazol, 5-Methyl-, 5-Ethyl-, 5-n-Propyl-, 5-Propylthio-, 5-iso-Propyl-, 5-tert-Butyl-, 5-Brom-, 5-Methylsulfinyl-, 5-Ethylsulfinyl-, 5-Propylsulfinyl-, 5-Methylsulfonyl-, 5-Ethylsulfonyl-, 5-Propyl-sulfonyl-, 5-Methoxycarbonyl-, 5-Ethoxy-carbonyl-, 5-(1-Cyano-2-methyl-propyl)-, 5-Benzyloxymethyl-, 5-Acetylamino-, 5-Nitro-, 5-Propylthio-, 5-Trifluormethyl-, 5-Trichlormethyl-, 5-Methylamino- und 5-(N-Methyl-N-tert-butylcarbonyl-amino)-2-chlor-1,3,4-oxadiazol, -2-brom-1,3,4-oxadiazol, -2-chlor-1,3,4-thiadiazol und -2-brom-1,3,4-thiadiazol;
2-Chlor- und 2-Brom-benzoxazol, 2-Chlor- und 2-Brom-benzthiazol; 5-Methyl-2-chlor-benzoxazol, 2-Chlor-6-ethoxy-benzthiazol, 2,5-Dichlor-benzoxazol, 2-Chlor-6-trifluormethyl-benzthiazol, 2-Chlor-5-trifluormethyloxy-benzthiazol, 2-Chlor-5,6-difluormethylendioxy-benzthiazol, 2,4,6,7-Tetrachlorbenzthiazol, 2-Chlor-4,6-difluor-benzthiazol, 2-Chlor-5-nitro-benzthiazol, 2-Chlor-6-nitro-benzthiazol, 2-Chlor-5-nitro-benzoxazol, 2-Chlor-5-cyano-benzoxazol und 5-Chlor-1-phenyl(1H)tetrazol.

Halogenazole der Formel (III) sind bekannt (vergleiche Elderfield, Heterocyclic Compounds Vol. 5 (1957), S. 298 und S.452; Vol. 7 (1961), S. 463 und S. 541; Weissberger, The Chemistry of Heterocyclic Compounds, (a) 'Five-Membered Heterocyclic Compounds with Nitrogen and Sulfur or Nitrogen, Sulfur and Oxygen' (1952), S. 35 und S. 81, (b) 'Five and Six-Membered Compounds with Nitrogen and Oxygen' (1962), S. 5, S. 245 und S. 263; Advances in Heterocyclic Chemistry, Vol. 5 (1965), S. 119; Vol. 7 (1966), S. 183; Vol. 17 (1974), S. 99 und Vol. 2o (1976), S. 65; Synthesis 1978, 8o3; Tetrahedron Letters 1968, 829; Chem. Ber. 89 (1956), 1534; 9o (1957), 182; 92 (1959), 1928; J.org.Chem. 27 (1962), 2589; DE-OS 1 67o 7o6, 1 164 413 und 2 213 865; DE-AS 1 251 327; GB-PS 1 128o25).

Das erfindungsgemäße Verfahren wird unter Verwendung von aprotischen Verdünnungsmitteln durchgeführt. Als solche kommen vor allem folgende Gruppen von organischen Lösungsmitteln in Betracht: Aromatische, gegebenenfalls halogenierte Kohlenwasserstoffe, wie z.B. Benzol, Toluol, Xylol, Chlorbenzol und o-Dichlorbenzol; Ether, wie z.B. Diethylether, Dipropylether, Di-iso-propylether, Dibutylether, Di-isobutylether, Methyl-propylether, Methyl-isopropylether, Methylbutylether, Methyl-isobutylether, Tetrahydrofuran und Dioxan; Ketone wie z.B. Aceton, Methylethylketon, Methyl-propylketon, Methyl-isopropylketon, Methyl-butylketon und Methyl-isobutylketon; Carbonsäureester und Amide, wie z.B. Essigsäureethylester,Essigsäurepropylester, Essigsäurebutylester, Dimethylformamid und Dimethylacetamid; sowie Sulfoxide und Sulfone, wie z.B. Dimethylsulfoxid und Tetramethylensulfon (Sulfolan).

Das beim erfindungsgemäßen Verfahren als Säurebindemittel dienende Kaliumhydroxid wird in wasserfreier, fester Form eingesetzt.Vorzugsweise verwendet man Kaliumhydroxid-Schuppen oder Kaliumhydroxid-Plätzchen.

Die Reaktionstemperatur kann innerhalb eines größeren Bereichs variiert werden. Sie liegt im allgemeinen zwischen -5o und +5o°C, vorzugsweise zwischen -2o und +1o°C.

Zur Durchführung des erfindungsgemäßen Verfahrens setzt man je Mol Halogen-hetaren der Formel (III) im allgemeinen zwischen 1 und 2 Mol, vorzugsweise zwischen 1,o und 1,5 Mol Hydroxyacetamid der Formel (II) und zwischen 1 und 3 Mol, vorzugsweise zwischen 1,2 und 2,o Mol Kaliumhydroxid ein.

In einer bevorzugten Ausführungsform des erfindungsgemäßen Verfahrens wird das Kaliumhydroxid im Verdünnungsmittel vorgelegt und die Ausgangsstoffe der Formeln (II) und (III) werden gleichzeitig oder nacheinander [in diesem Fall (II) vor (III)] dazugegeben. Das Reaktionsgemisch wird bis zum Ende der Umsetzung gerührt.

Die Aufarbeitung kann nach üblichen Methoden durchgeführt werden, beispielsweise durch Verdünnen mit einem mit Wasser praktisch nicht mischbaren organischen Lösungsmittel, wie z.B. Toluol, Waschen mit verdünnter Salzsäure und dann mit Wasser, Abtrennen der organischen Phase und Abdestillieren des Lösungsmittels. Die zurückbleibenden Rohprodukte können bei Bedarf nach üblichen Methoden weiter gereinigt werden.

Die nach dem erfindungsgemäßen Verfahren herzustellenden Verbindungen der Formel (I) können als Herbizide verwendet werden (vergleiche EP 55o1 und 18 497 sowie DE-OS 2 822 155, 2 9o3 966, 2 914 oo3, 2 946 432, 2 946 524 und 3 oo4 326).

Beispiel 1

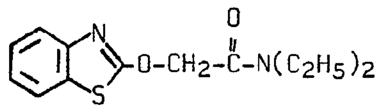

Zu einer auf 0 bis 5°C gekühlten Mischung aus 11,2 g (o,2 Mol) Kaliumhydroxid-Plätzchen (12,7 g 88%iges Produkt) und 5o ml Toluol werden 19,7 g (o,15 Mol) Hydroxyessigsäure-N,N-diethylamid tropfenweise gegeben und das Gemisch wird 30 Minuten bei 0 bis 5°C gerührt. Dann werden 17 g (o,1 Mol) 2-Chlor-benzthiazol (gelöst in 5o ml Toluol) zugetropft und das Reaktionsgemisch wird weitere 3 Stunden bei 0 bis 5°C gerührt. Man gibt dann soviel Toluol dazu, daß eine klare Lösung entsteht, wäscht mit 2oo ml verdünnter Salzsäure und dann mit Wasser (2 x 25o ml) und destilliert das Lösungsmittel unter vermindertem Druck ab. Man erhält 26,4 g (1oo% der Theorie) 2-Benzthiazolyl-oxyessigsäure-N,N-diethylamid vom Schmelzpunkt 62-63°C.

Beispiel 2

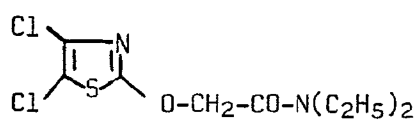

Zu einer auf -15°C gekühlten Mischung aus 16,8 g (o,3 Mol) Kaliumhydroxid-Plätzchen (19,1 g 88%iges Produkt) und 1oo ml Methylisobutylketon werden 37,8g (o,2 Mol) 2,4,5-Trichlorthiazol und 28,8 g (o,22 Mol) Hydroxyessigsäure-N,N-diethylamid tropfenweise zugegeben. Das Gemisch wird 3o Minuten bei -15°C und anschließend 1 Stunde bei -5°C gerührt. Man gibt 2oo ml Methylisobutylketon zu, wäscht mit 15o ml verdünnter Salzsäure und dann mit Wasser (2 x 25o ml). Das Lösungsmittel wird unter vermindertem Druck abdestilliert. Man erhält 54,9 g (97% der Theorie) 4,5-Dichlor-2-thiazolyl-oxyessigsäure-N,N-diethylamid vom Schmelzpunkt 44-45°C.

Beispiel 3

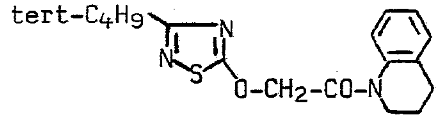

Zu einer auf -5°C gekühlten Mischung aus 44,9 g (0,8 Mol) Kaliumhydroxid-Plätzchen (51 g 88 %iges Produkt) und 200 ml Tetrahydrofuran werden 89,4 g (0,5 Mol) 3-tert.-Butyl-5-chlor-1,2,4-thiadiazol und 95,7 g (0,5 Mol) Hydroxy-essigsäure-N-(1,2,3,4-tetrahydrochinolid), gelöst in 500 ml Tetrahydrofuran, unter heftigem Rühren zugetropft. Das Reaktionsgemisch wird 2 Stunden bei -5°C und nach Erwärmen auf 20°C noch eine Stunde bei 20°C nachgerührt. Man gibt 300 ml Xylol zu und wäscht mit 250 ml Wasser. Die wäßrige Phase wird anschließend mit Xylol extrahiert. Die vereinigten organischen Phasen werden mit verdünnter Salzsäure und dann mit Wasser gewaschen. Das Lösungsmittel wird unter vermindertem Druck entfernt. Man erhält 155 g (86 %der Theorie) 3-tert.-Butyl-5-(1,2,4-thiadiazolyl)-oxy-essigsäure-N-(1,2,3,4-tetrahydrochinolid) vom Schmelzpunkt 66-67°C.

In analoger Weise können die nachfolgenden Verbindungen erhalten werden:

Beispiel 4

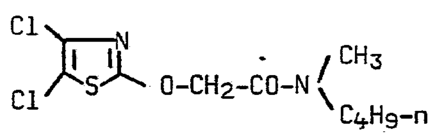

Schmelzpunkt 41-42°C

Beispiel 5

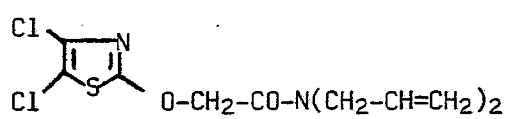

Schmelzpunkt 44-45°C

Beispiel 6

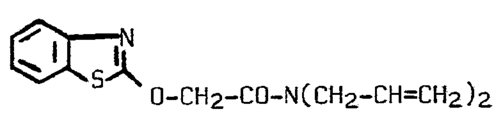

Schmelzpunkt 73-74°C

Patentansprüche

1. Verfahren zur Herstellung von Hetaryloxyacetamiden der allgemeinen Formel I

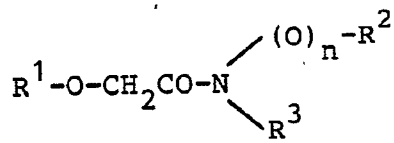

(I)

in welcher

$R^1$ für einen fünfgliedrigen Heterocyclus steht, welcher ein Sauerstoff- oder ein Schwefelatom und zusätzlich 1 bis 3 Stickstoffatome enthält und welcher gegebenenfalls substituiert ist durch Halogen, Cyano, Nitro, Amino, Alkylamino, Arylamino, Dialkylamino, Alkylcarbonylamino, Alkylcarbonyl, Carboxy, Alkoxycarbonyl, Carbamoyl, Alkylaminocarbonyl, Dialkylaminocarbonyl, gegebenenfalls durch Halogen, Nitro oder Alkyl substituiertes Arylaminocarbonyl, gegebenenfalls durch Halogen, Cyano, Nitro, Alkyl oder Alkoxy substituiertes Aryl, gegebenenfalls durch Halogen substituiertes Aralkyl, gegebenenfalls durch Halogen substituiertes Alkoxy, Alkenoxy, Alkinoxy, Alkoxycarbonylalkoxy, Aralkoxy oder Aryloxy, gegebenenfalls durch Halogen substituiertes Alkylthio, Alkenylthio, Alkinylthio, Alkoxycarbonylalkylthio, Aralkylthio oder Arylthio, gegebenenfalls durch Halogen substituiertes Alkylsulfinyl oder Alkylsulfonyl, gegebenenfalls durch Halogen substituiertes Alkyl, Alkenyl, Alkinyl, Alkoxyalkyl, Aralkoxyalkyl, Aryloxyalkyl, Alkylthioalkyl, Alkylsulfinylalkyl, Alkylsulfonylalkyl, Arylthioalkyl, Arylsulfinylalkyl, Arylsulfonylalkyl, Carboxyalkyl, oder Alkoxycarbonylalkyl, gegebenenfalls substituiertes Aminocarbonylalkyl, Cyanoalkyl oder Cycloalkyl, oder welcher gegebenenfalls benzanneliert ist, wobei der Benzo-Rest gegebenenfalls durch Halogen, Alkyl, Halogenalkyl, Alkoxy, Halogenalkoxy, Alkylthio, Halogenalkylthio, Amino, Alkylamino, Dialkylamino, Nitro, Cyano, Alkoxycarbonyl oder durch gegebenenfalls halogen-substituiertes Alkylendioxy substituiert ist,

oder in welcher

$R^1$ für einen durch Phenyl substituierten Tetrazolylrest steht, wobei der Phenylrest gegebenenfalls durch Halogen, Cyano, Nitro und/oder durch einen gegebenenfalls halogen-substituierten Rest aus der Reihe Alkyl, Alkoxy, Alkylthio und Alkylendioxy substituiert ist,

in welcher weiter

n für Null oder 1 steht und

$R^2$ und $R^3$, welche gleich oder verschieden sein können, einzeln für gegebenenfalls substituierte Reste aus der Reihe Alkyl, Alkenyl, Alkinyl, Cycloalk(en)yl, Aralkyl oder Aryl stehen oder - für den Fall, daß n für Null steht - zusammmen mit dem Stickstoffatom, an das sie gebunden sind, einen gegebenenfalls substituierten, gegebenenfalls teilweise ungesättigten und gegebenenfalls benzannellierten Mono- oder Bicyclus bilden, der gegebenenfalls weitere Heteroatome enthält,

dadurch gekennzeichnet, daß man Hydroxyacetamide der Formel II

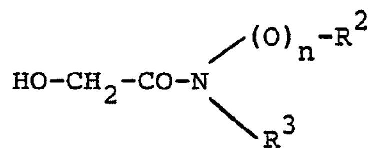
(II)

in welcher

n, $R^2$ und $R^3$ die oben angegebene Bedeutung haben,

mit Halogen-hetarenen der Formel III

$$R^1\text{-Hal} \qquad \text{(III)}$$

in welcher

$R^1$ die oben angegebene Bedeutung hat und

Hal für Fluor, Chlor, Brom oder Iod steht,

in Gegenwart von festem, wasserfreiem Kaliumhydroxid und in Gegenwart eines aprotischen Verdünnungsmittels bei Temperaturen zwischen -50 und +50°C umsetzt.

2. Verfahren nach Anspruch 1, dadurch gekennzeichnet, daß man bei Temperaturen zwischen -20 und +10°C arbeitet.

3. Verfahren nach Anspruch 1, dadurch gekennzeichnet, daß man auf 1 Mol Halogen-hetaren (III) 1-2 Mol, vorzugsweise 1-1,5 Mol Hydroxyacetamid (II) einsetzt.

4. Verfahren nach Anspruch 1, dadurch gekennzeichnet, daß man auf 1 Mol Halogen-hetaren (III) 1-3 Mol, vorzugsweise 1,2-2 Mol Kaliumhydroxid einsetzt.

5. Verfahren nach Anspruch 1, dadurch gekennzeichnet, daß man als aprotisches Verdünnungsmittel einen gegebenenfalls chlorierten Kohlenwasserstoff, einen Ether, ein Dialkylketon, einen Carbonsäurealkylester, ein Carbonsäureamid, ein Sulfoxid oder ein Sulfon verwendet.

6. Verfahren nach Anspruch 1, dadurch gekennzeichnet, daß man als Hydroxyacetamid der allgemeinen Formel (II) Hydroxyessigsäure-N,N-diethylamid und als Halogen-hetaren der allgemeinen Formel (III) 2,4,5-Trichlorthiazol einsetzt.

7. Verfahren nach Anspruch 1, dadurch gekennzeichnet, daß man das Kaliumhydroxid im aprotischen Verdünnungsmittel vorlegt und die Ausgangsstoffe der Formeln (II) und (III) gleichzeitig oder nacheinander - in diesem Falle (II) vor (III) - unter Rühren hinzufügt.

Europäisches Patentamt

# EUROPÄISCHER RECHERCHENBERICHT

0081730

Nummer der Anmeldung

EP 82 11 0970

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 018 497 (BAYER AG) * Seiten 26-28, Herstellungsbeispiele * | 1 | C 07 D 277/34<br>C 07 D 277/68<br>C 07 D 417/12<br>C 07 D 257/04<br>C 07 D 263/38<br>C 07 D 263/58<br>C 07 D 271/06<br>C 07 D 271/10<br>C 07 D 285/08<br>C 07 D 285/12 |
| | --- | | |
| A | EP-A-0 037 938 (NIHON TOKUSHU NOYAKU SEIZO K.K.) * Seiten 4,5; Seite 16, Beispiel 1 * | 1 | |
| | --- | | |
| A | TETRAHEDRON LETTERS, Band 35, Nr. 18, 1979, Seiten 2169-2173, Pergamon Press Ltd., Oxford, G.B. R.A.W. JOHNSTONE et al.: "A rapid, simple, and mild procedure for alkylation of phenols, alcohols, amides and acids" * Insgesamt * | 1 | RECHERCHIERTE SACHGEBIETE (Int. Cl. 3) |
| | --- | | |
| P | DE-A-3 038 636 (BAYER AG) * Ansprüche * | 1 | C 07 D 277/00<br>C 07 D 263/00<br>C 07 D 285/00<br>C 07 D 257/00<br>C 07 D 271/00 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 25-02-1983 | HENRY J.C. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82